# EUROPEAN PATENT APPLICATION

(11) **EP 4 113 425 A1**
(43) Date of publication of application: **04.01.2023**
(21) Application number: 21759828.3
(22) Date of filing: 26.02.2021
(51) Int. Cl.: G06Q 50/10, A61M 21/02, G16H 50/30

(54) **WEARABLE APPLIANCE, INFORMATION PROCESSING DEVICE, INFORMATION PROCESSING SYSTEM, AND PROGRAM**

(30) Priority: 28.02.2020 JP 2020034124
(71) Applicant: Astellas Pharma Inc., Chuo-ku Tokyo 103-8411 (JP)
(72) Inventor: AOKI, Toshiaki, Tokyo 103-8411 (JP); NAKAO, Yuko, Tokyo 103-8411 (JP); ENOMOTO, Ryugo, Tokyo 103-8411 (JP); HARAGUCHI, Akiko, Tokyo 103-8411 (JP)
(74) Representative: V.O.
(86) International application number: PCT/JP2021/007486
(87) International publication number: WO 2021/172553

(57) **Abstract**

A detection unit (88) detects a specific state of a wearer. A signal communication unit (92) wirelessly transmits, to an information processing apparatus, log information indicating a detection result obtained by the detection unit (88) detecting the specific state and a date and time when the specific state is detected. A stimulus application unit (94) applies a stimulus to the wearer when the specific state is detected by the detection unit (88).

## Description

### Technical Field

The technology of the present disclosure relates to a wearable device, an information processing apparatus, an information processing system, and a program.

### Background Art

Conventionally, a wearable device capable of changing a heart rate of a person by providing a stimulus impulse is known (see, for example, Patent Literature 1). In this wearable device, a rhythmic tactile stimulus that can change the heart rate or other physiological parameters of a user is given to the user, and information such as rhythm of the tactile stimulus is wirelessly transmitted to the outside for learning.

### Citation List

### Patent Literature

Patent Literature 1: WO 2015/118302 A

### SUMMARY OF INVENTION

### Technical Problem

Patent Literature 1 does not describe that an emotional state of a person is logged and recorded. It is not possible to prompt the person to look back on the state later.

An object of the technology of the disclosure is to provide a wearable device capable of logging detection of a specific state of a wearer and applying a stimulus to the wearer in the specific state.

Another object of the disclosure is to provide an information processing apparatus, an information processing system, and a program capable of logging detection of a specific state of a wearer, prompting input of a record regarding the detection of the specific state, and giving an opportunity to objectively look back on the specific state of the wearer on the basis of the log information.

### Solution to Problem

A first aspect of the disclosure is a wearable device including: a detection unit that detects a specific state of a wearer; a signal communication unit that wirelessly transmits, to an information processing apparatus, log information indicating a detection result obtained by the detection unit detecting the specific state; and a stimulus application unit that applies a stimulus to the wearer when the specific state is detected by the detection unit.

A second aspect of the disclosure is an information processing apparatus including: a communication unit that wirelessly receives, from a wearable device, log information indicating detection of a specific state of a wearer; a display unit that displays a message for prompting the wearer to make an input regarding the detection of the specific state indicated by the log information; an input reception unit that receives the input by the wearer with respect to the detection of the specific state indicated by the log information; and a recording unit that records the log information and the input by the wearer.

A third aspect of the disclosure is an information processing system including: the wearable device; and the information processing apparatus. A communication unit of the information processing apparatus receives the log information from the wearable device.

A fourth aspect of the disclosure is a program for causing a computer to function as: a communication unit that wirelessly receives, from a wearable device, log information indicating detection of a specific state of a wearer; a display unit that displays a message for prompting the wearer to make an input regarding the detection of the specific state indicated by the log information; an input reception unit that receives the input by the wearer with respect to the detection of the specific state indicated by the log information; and a recording unit that records the log information and the input by the wearer.

A fifth aspect of the disclosure is an information processing apparatus including: a first input reception unit that receives, from a user, input information indicating that the user is in a specific state; a display unit that displays a message for prompting the user to make an input regarding the specific state indicated by the input information; a second input reception unit that receives the input by the user with respect to the specific state indicated by the input information; and a recording unit that records the input information and the input by the user.

A sixth aspect of the disclosure is a program for causing a computer to function as: a first input reception unit that receives, from a user, input information indicating that the user is in a specific state; a display unit that displays a message for prompting the user to make an input regarding the specific state indicated by the input information; a second input reception unit that receives the input by the user with respect to the specific state indicated by the input information; and a recording unit that records the input information and the input by the user. Advantageous Effects of Invention

According to the wearable device according to the technology of the disclosure, it is possible to log the detection of the specific state of the wearer and the detection date and time thereof and to apply the stimulus to the wearer in the specific state.

According to the information processing apparatus, the information processing system, and the program according to the technology of the disclosure, it is possible to log the occurrence of the specific state of the wearer, the detection date and time thereof, or the like and to prompt the input regarding the occurrence of the specific state and give an opportunity to look back each time. Furthermore, the aggregation result of the log can also be used to look back.

### BRIEF DESCRIPTION OF DRAWINGS

Fig. 1 is a schematic diagram illustrating a configuration of an information processing system according to a first embodiment of a technology of the disclosure.
Fig. 2(A) is a side view illustrating the wearable device according to the first embodiment of the technology of the disclosure, and Fig. 2(B) is a front view illustrating the wearable device according to the first embodiment of the technology of the disclosure.
Fig. 3 is a block diagram illustrating a configuration of the wearable device of the information processing system according to the first embodiment of the technology of the disclosure.
Fig. 4 is a block diagram illustrating a configuration of a mobile information terminal of the information processing system according to the first embodiment of the technology of the disclosure.
Fig. 5 is a block diagram illustrating a configuration of a control unit of the mobile information terminal of the information processing system according to the first embodiment of the technology of the disclosure.
Fig. 6 is a diagram illustrating an example of an input screen.
Fig. 7 is a flowchart illustrating contents of a log-information transmission processing routine of the wearable device according to the first embodiment of the technology of the disclosure.
Fig. 8 is a flowchart illustrating contents of a log-information recording processing routine of the mobile information terminal according to the first embodiment of the technology of the disclosure.
Fig. 9 is a flowchart illustrating contents of an input recommendation notification processing routine of the mobile information terminal according to the first embodiment of the technology of the disclosure.
Fig. 10 is a schematic diagram illustrating a configuration of an information processing system according to a second embodiment of the technology of the disclosure.
Fig. 11 is a block diagram illustrating a configuration of a mobile information terminal of the information processing system according to the second embodiment of the technology of the disclosure.
Fig. 12 is a block diagram illustrating a configuration of a control unit of a mobile information terminal of an information processing system according to a third embodiment of the technology of the disclosure.
Fig. 13 is a diagram illustrating an example of an emotion input screen.
Fig. 14 is a diagram illustrating an example of a moving object operation screen.
Fig. 15 is a flowchart illustrating contents of a log-information recording processing routine of the mobile information terminal according to the second embodiment of the technology of the disclosure.

### DESCRIPTION OF EMBODIMENTS

Hereinafter, embodiments of the technology of the disclosure will be described in detail with reference to the drawings.

### [First Embodiment]

In a first embodiment, a case where the technology of the disclosure is applied to an information processing system for logging detection of an emotion of anger of a wearer wearing a bracelet type wearable device will be described as an example.

### <Configuration of Information Processing System 10>

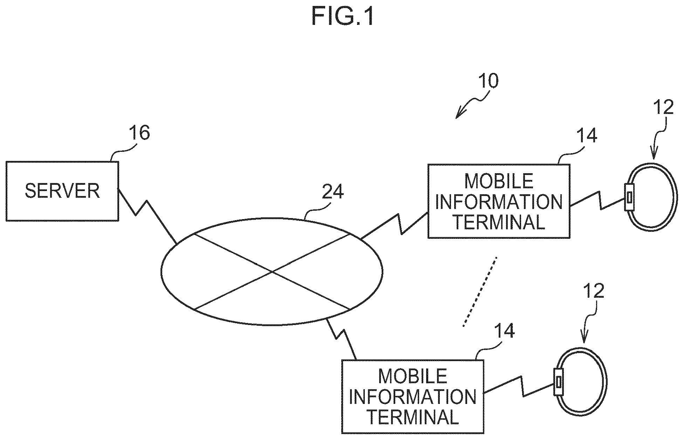

As illustrated in Fig. 1, an information processing system 10 according to the first embodiment of the technology of the disclosure includes a plurality of wearable devices 12, a plurality of mobile information terminals 14 carried by a plurality of wearers wearing the plurality of wearable devices, and a server 16.

The mobile information terminal 14 and the server 16 are connected via a network 24 such as the Internet. In addition, the wearable device 12 and the mobile information terminal 14 are connected by wireless communication such as Bluetooth (registered trademark).

### <Configuration of Wearable Device 12>

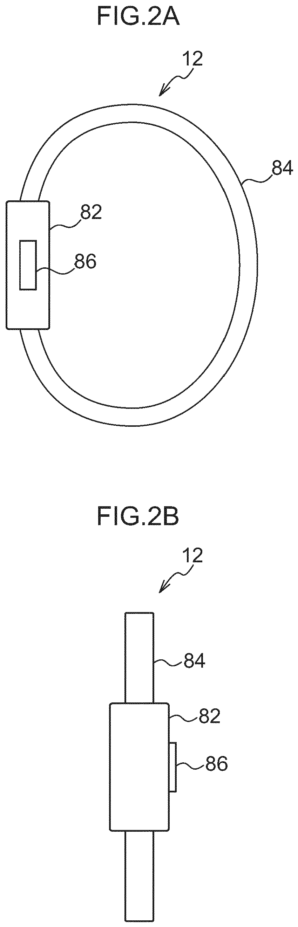

As illustrated in Figs. 2(A) and 2(B), the wearable device 12 includes a wearable device body 82 and an elongated band-shaped wearing part 84 to be worn on an arm of a wearer.

A button type or pressure sensor type switch 86 pressed by the wearer is provided on a side surface or a surface of the wearable device body 82. The switch 86 is pressed when the wearer becomes aware of the emotion of anger.

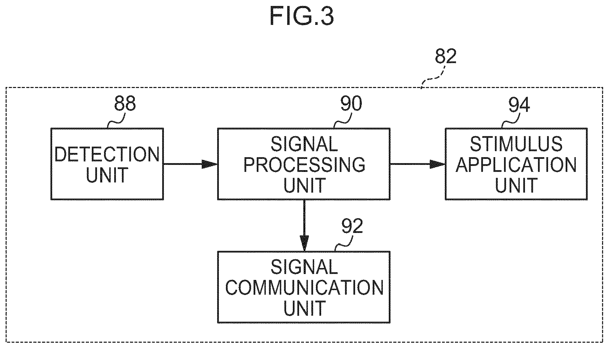

As illustrated in Fig. 3, the wearable device body 82 further includes a detection unit 88, a signal processing unit 90, a signal communication unit 92, and a stimulus application unit 94.

The detection unit 88 detects that the button type or pressure sensor type switch 86 is pressed by the wearer who becomes aware of the emotion of anger.

In a case where the detection unit 88 detects that the button type or pressure sensor type switch 86 is pressed, the signal processing unit 90 outputs a signal indicating that the switch 86 is pressed to the stimulus application unit 94. In addition, in a case where the detection unit 88 detects that the switch 86 is pressed, the signal processing unit 90 records the pressing of the switch 86 and the date and time of the pressing as log information, and outputs the log information to the signal communication unit 92.

When the log information output from the signal processing unit 90 is input, the signal communication unit 92 transmits the log information indicating the detection of the emotion of anger of the wearer and the detection date and time to the mobile information terminal 14 via wireless communication.

When the signal which is output from the signal processing unit 90 and indicates that the button type or pressure sensor type switch 86 has been operated is input, the stimulus application unit 94 applies a vibration stimulus to the wearing portion of the arm of the wearer. For example, an actuator provided in the wearable device body 82 applies a predetermined vibration stimulus to the wearing portion of the arm of the wearer.

### <Configuration of Mobile Information Terminal 14>

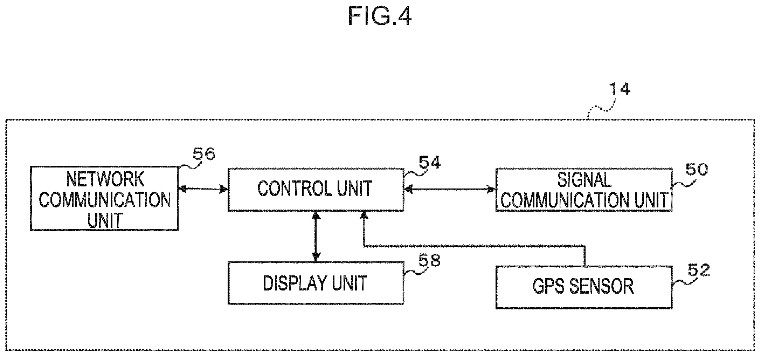

The mobile information terminal 14 is, for example, a smartphone, and includes a signal communication unit 50, a GPS sensor 52, a control unit 54, a network communication unit 56, and a display unit 58 as illustrated in Fig. 4.

The signal communication unit 50 transmits and receives signals to and from the wearable device 12 by wireless communication.

The GPS sensor 52 measures position information of the mobile information terminal 14.

The network communication unit 56 transmits and receives data via the network 24.

The display unit 58 includes, for example, a touch panel display, and displays not only an input screen regarding the log information but also a screen displaying a result of aggregation of the log information.

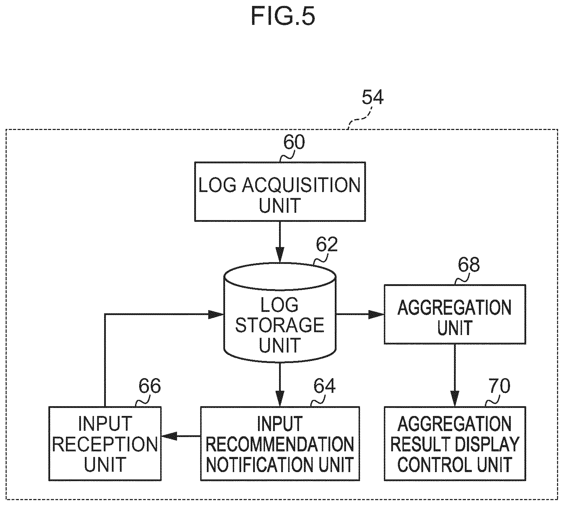

The control unit 54 includes a computer including a CPU, a ROM, a RAM, and an HDD, and the HDD stores application software which is downloaded from a site provided by a web server (not illustrated) and installed and includes a log-information recording processing routine and an input recommendation notification processing routine to be described later. The control unit 54 is functionally configured as follows. As illustrated in Fig. 5, the control unit 54 includes a log acquisition unit 60, a log storage unit 62, an input recommendation notification unit 64, an input reception unit 66, an aggregation unit 68, and an aggregation result display control unit 70.

In a case where the log information is received by the signal communication unit 50, the log acquisition unit 60 acquires the log information, further acquires a current position measured by the GPS sensor 52, adds the current position to the received log information, and stores the log information in the log storage unit 62.

The log storage unit 62 stores the log information in association with flag information indicating whether the input regarding the detection of the emotion of anger by the wearer of the wearable device 12 is received. In addition, the log storage unit 62 stores the input information of the wearer received regarding the detection of the emotion of anger in association with the log information.

In a case where the log information associated with the flag information indicating that the input from the wearer is not yet received is stored in the log storage unit 62, the input recommendation notification unit 64 causes the display unit 58 to display a notification for prompting the wearer to make an input.

In a case where there is an input instruction indicating that the wearer performs the input regarding the detection of the emotion of anger, the input reception unit 66 causes the display unit 58 to display an input screen regarding the log information, and receives the input information regarding the detection of the emotion of anger. The input reception unit 66 stores the received input information in the log storage unit 62 in association with the log information. At this time, the flag information for the log information is updated to flag information indicating that the input by the wearer is received.

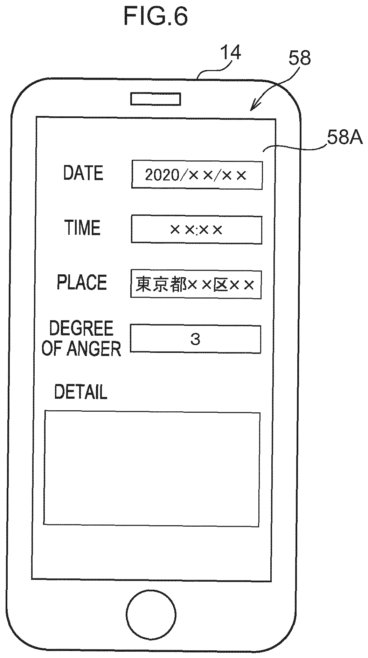

For example, an input screen 58A as illustrated in Fig. 6 is displayed on the display unit 58. On this input screen, the date, the time, and the place in which the emotion of anger is detected are displayed on the basis of the log information, and the input of the degree of the emotion of anger and detailed information regarding the emotion of anger is received. As the degree of the emotion of anger, the input of a score indicating the intensity, the characteristics, or the like of the emotion of anger is received, and as the detailed information regarding the emotion of anger, the input of text information indicating the cause of the emotion of anger and the situation, the feeling, and the like of the wearer at the time of occurrence of the emotional state of anger is received.

On the basis of the log information and the input information stored in the log storage unit 62, the aggregation unit 68 obtains an aggregation result regarding each of the date, the time, and the place in which the emotion of anger is detected and the degree of the emotion of anger. For example, a frequency at which the emotion of anger is detected for each date, a frequency at which the emotion of anger is detected for each time zone, a frequency at which the emotion of anger is detected for each place, a frequency at which the emotion of anger is detected for each degree of the emotion of anger, an input frequency of the text information, an appearance frequency of an input word regarding an emotion in the text information, and the like are obtained as the aggregation result.

The aggregation result display control unit 70 causes the display unit 58 to display a screen which visualizes the aggregation result regarding each of the date, the time, the place, and the degree of the emotion of anger obtained by the aggregation unit 68.

The aggregation unit 68 may transmit the obtained aggregation result to the server 16. In this case, the server 16 may obtain the aggregation result of all the wearers on the basis of the aggregation result transmitted from each mobile information terminal 14, and transmit the aggregation result to each mobile information terminal 14. Furthermore, the aggregation result of all the wearers may be displayed on the display unit 58 of each mobile information terminal 14.

### <Operation of Information Processing System 10>

Next, an operation of the information processing system 10 according to the first embodiment of the technology of the disclosure will be described.

First, when the wearer wearing the wearable device 12 becomes aware of the emotion of anger, the wearer presses the switch 86 of the wearable device 12.

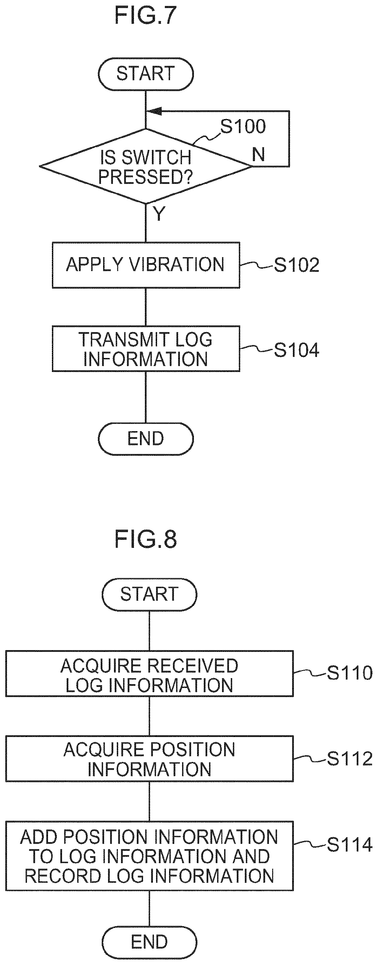

At this time, the wearable device body 82 of the wearable device 12 executes the log-information transmission processing routine illustrated in Fig. 7.

First, in step S100, the signal processing unit 90 determines whether the detection unit 88 detects the pressing of the switch 86. When it is determined that the detection unit 88 detects the pressing of the switch 86, the process proceeds to step S102.

In step S102, the stimulus application unit 94 applies a vibration stimulus to the wearing portion of the arm of the wearer.

In step S104, the signal communication unit 92 transmits log information indicating the detection of the emotion of anger of the wearer to the mobile information terminal 14 via wireless communication, and ends the log-information transmission processing routine. Note that the order of the processing in step S102 and the processing in step S104 may be exchanged, or the processing in step S102 and the processing in step S104 may be performed in parallel.

Then, when receiving the log information from the wearable device 12, the mobile information terminal 14 executes the log-information recording processing routine illustrated in Fig. 8.

First, in step S110, the log acquisition unit 60 acquires the log information received by the signal communication unit 50.

In step S112, the log acquisition unit 60 acquires the current position measured by the GPS sensor 52. Then, in step S114, the log acquisition unit 60 adds the current position to the received log information. The log acquisition unit 60 stores the log information in the log storage unit 62 in association with the flag information indicating that the input regarding the detection of the emotion of anger by the wearer of the wearable device 12 is not received.

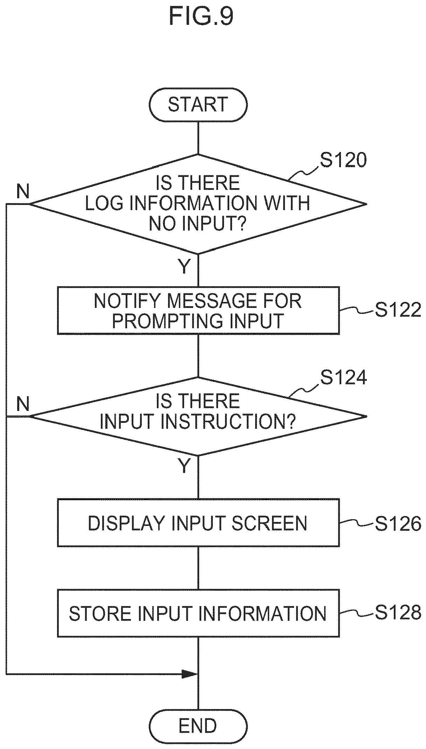

Then, the mobile information terminal 14 periodically executes the input recommendation notification processing routine illustrated in Fig. 9.

First, in step S120, the input recommendation notification unit 64 determines whether the log information associated with the flag information indicating that the input by the wearer is not received is stored in the log storage unit 62. In a case where there is no log information associated with the flag information indicating that the input by the wearer is not received, the input recommendation notification processing routine is ended. On the other hand, in a case where there is the log information associated with the flag information indicating that the input by the wearer is not received, in step S122, the input recommendation notification unit 64 causes the display unit 58 to display a notification for prompting the wearer to make an input regarding the log information.

In step S124, the input reception unit 66 determines whether there is an input instruction indicating that the wearer performs the input regarding the detection of the emotion of anger. In a case where there is not the input instruction indicating that the wearer performs the input regarding the detection of the emotion of anger, the input recommendation notification processing routine is ended. On the other hand, in a case where there is the input instruction indicating that the wearer performs the input regarding the detection of the emotion of anger, in step S126, the input reception unit 66 causes the display unit 58 to display an input screen regarding the log information. The input reception unit 66 receives the input information regarding the detection of the emotion of anger. In step S128, the input reception unit 66 stores the received input information in the log storage unit 62 in association with the log information. At this time, the flag information for the log information is updated to flag information indicating that the input by the wearer is received.

Then, the mobile information terminal 14 receives, from the wearer, an instruction to display the aggregation result regarding each of the date, the time, and the place in which the emotion of anger is detected, and the degree of the emotion of anger. At this time, on the basis of the log information and the input information stored in the log storage unit 62, the aggregation unit 68 obtains an aggregation result regarding each of the date, the time, and the place in which the emotion of anger is detected and the degree of the emotion of anger. Then, the aggregation result display control unit 70 causes the display unit 58 to display a screen that visualizes the aggregation result regarding each of the date, the time, the place, and the degree of the emotion of anger obtained by the aggregation unit 68. In addition, the aggregation result display control unit 70 may display the date and time when the wearer feels angry on a calendar.

As described above, the information processing system according to the first embodiment of the technology of the disclosure logs the detection of the emotional state of anger of the wearer, prompts the wearer to input the record regarding the detection of the emotional state of anger, and further aggregates the emotional state of anger on the basis of the log information and the input information. The information processing system can give an opportunity to objectively look back on the own specific state on the basis of the information and the aggregation result. In particular, it is possible to control an emotion by allowing the input of a cause of emotional disturbance, his/her feeling at that time, and the intensity of the emotion of anger to give the opportunity to look back.

According to the wearable device, it is possible to log the detection of the emotional state of anger of the wearer and to apply a stimulus to the wearer in the emotional state of anger. In particular, when the wearer presses the button type or pressure sensor type switch, it is possible to switch off the emotion of anger, and it is possible to distract the mind on the spot by the vibration stimulus to control the emotional disturbance.

By storing the input information for the log information and looking back on the log information and the input information at a later date, the cognitive distortion can be eliminated to control the emotional disturbance of anger.

By controlling irritation/anger, a relationship with surrounding people is improved, and an interpersonal relationship and a social relationship become positive. In addition, by objectively reflecting and controlling oneself who is irritated and angry, it is possible to interact more positively with oneself.

Note that, in the above-described embodiment, a case where the information processing system is used to control the emotional state of anger has been described as an example, but the present invention is not limited thereto. For example, the technology of the disclosure may be applied to an information processing system for controlling the emotional state of anxiousness. In addition, the technology of the disclosure may be applied to an information processing system for controlling an impulsive emotional state such as irritation, panic, suicidal ideation, or catastrophic thoughts. In addition, the technology of the disclosure may be applied to an information processing system for controlling a specific state other than the emotional state.

A case where the wearer inputs the degree of the emotional state of anger on the input screen has been described as an example, but the present invention is not limited thereto, and the wearable device itself may detect the degree of the emotional state of anger. The log information including the degree of the emotional state of anger detected, for example, on the basis of the pressing time, the pressing frequency, the pressing force, or the like of the button type or pressure sensor type switch may be transmitted to the information processing apparatus.

A case where the wearable device is a bracelet type has been described as an example, but the present invention is not limited thereto, and the technology of the disclosure may be applied to a ring type wearable device, a smartwatch type wearable device, a necklace type wearable device, an earphone type wearable device, a headset type wearable device, a spectacle type wearable device, or a wearable device to be attached on the skin.

A case where the wearable device applies a vibration stimulus to the wearer has been described as an example, but the present invention is not limited thereto, and the wearable device may apply a temperature stimulus, an electrical stimulus, a sound wave stimulus, or an odor stimulus to the wearer.

The vibration time, the vibration frequency, the vibration intensity, and the like of the wearable device may be adjusted by the information processing apparatus according to the degree of the emotional state of anger of the wearer, or the like.

The wearable device itself may detect information such as a heartbeat, an electrodermal activity, a myoelectric potential, a movement of a muscle such as a respiratory muscle, an electroencephalogram, or a physical activity amount, and analyze the detected information, predict the emotion of the wearer, and the wearable device may automatically apply a vibration stimulus to the wearer according to the predicted emotion.

A case where the wearer presses the switch of the wearable device when the wearer wearing the wearable device becomes aware of the emotion of anger has been described as an example, but the present invention is not limited thereto. For example, when the wearer wearing the wearable device becomes aware of the emotion of anger, the wearer may grip the wearable device with the whole arm. By gripping the wearable device with the whole arm, the pressure sensor type switch on the wearable device is pressed, and as a result, a vibration stimulus is applied to the wearer.

### [Second Embodiment]

In a second embodiment, a case where the technology of the disclosure is applied to an information processing system for logging the occurrence of a specific emotion of a user of the mobile information terminal 14 will be described as an example. Note that the portions having the same configurations as those of the first embodiment are denoted by the same reference numerals, and the description thereof is omitted.

### <Configuration of Information Processing System 200>

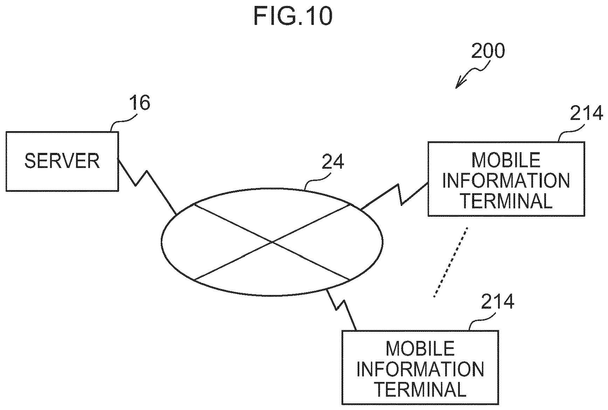

As illustrated in Fig. 10, an information processing system 200 according to the second embodiment of the technology of the disclosure includes a plurality of mobile information terminals 214 carried by a plurality of users, and the server 16.

The mobile information terminal 214 and the server 16 are connected via the network 24 such as the Internet.

### <Configuration of Mobile Information Terminal 214>

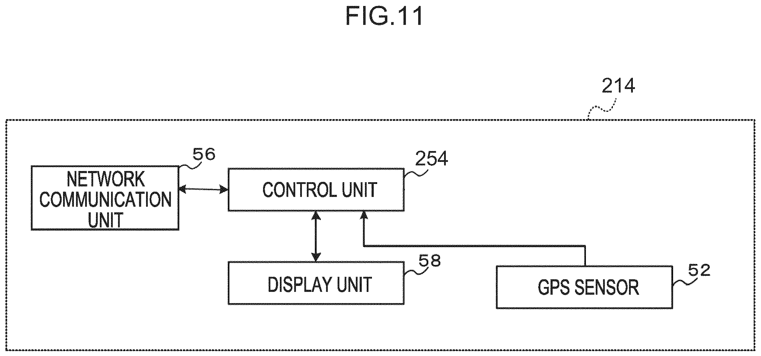

The mobile information terminal 214 is, for example, a smartphone, and includes the GPS sensor 52, the control unit 254, the network communication unit 56, and the display unit 58 as illustrated in Fig. 11.

The display unit 58 includes, for example, a touch panel display, and displays not only an input screen regarding the log information but also a screen displaying a result of aggregation of the log information. In addition, the display unit 58 further displays an emotion input screen for inputting the emotion of the user and a moving object operation screen for receiving an operation of tracking a moving object on the screen.

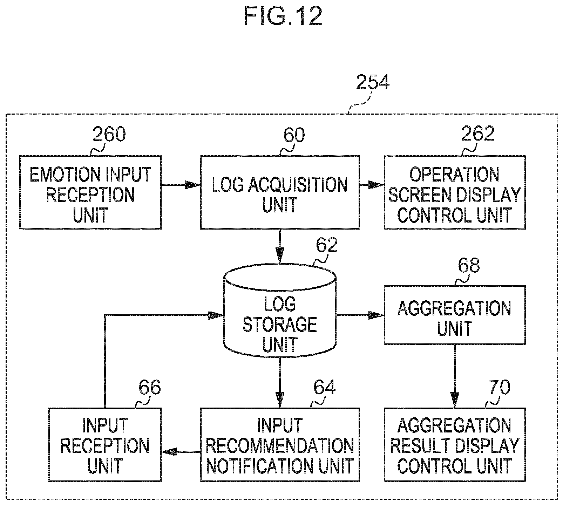

The control unit 254 includes a computer including a CPU, a ROM, a RAM, and an HDD, and the HDD stores application software which is downloaded from a site provided by a web server (not illustrated) and installed and includes a log-information recording processing routine and an input recommendation notification processing routine to be described later. The control unit 254 is functionally configured as follows. As illustrated in Fig. 12, the control unit 254 includes an emotion input reception unit 260, a log acquisition unit 60, an operation screen display control unit 262, the log storage unit 62, the input recommendation notification unit 64, the input reception unit 66, the aggregation unit 68, and the aggregation result display control unit 70. Note that the emotion input reception unit 260 is an example of a first input reception unit, and the input reception unit 66 is an example of a second input reception unit.

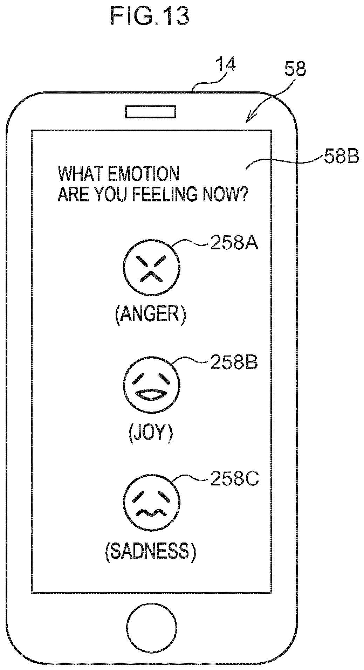

In a case where there is an input instruction for the user to input the occurrence of a specific emotion, the emotion input reception unit 260 causes the display unit 58 to display an emotion input screen 58B illustrated in Fig. 13 and receives the input indicating the occurrence of the specific emotion. Fig. 13 illustrates an example in which the emotion input screen 58B displays a mark 258A indicating an emotion of anger, a mark 258B indicating an emotion of joy, and a mark 258C indicating an emotion of sadness, and the selection of the type of emotion is received when the user performs a touch operation on the marks 258A to 258C.

When the type of emotion is selected on the emotion input screen 58B, the emotion input reception unit 260 outputs log information indicating the occurrence of the selected type of emotion and the detection date and time thereof to the log acquisition unit 60.

In a case where the log information is input from the emotion input reception unit 260, the log acquisition unit 60 acquires the log information, further acquires a current position measured by the GPS sensor 52, adds the current position to the received log information, and stores the log information in the log storage unit 62.

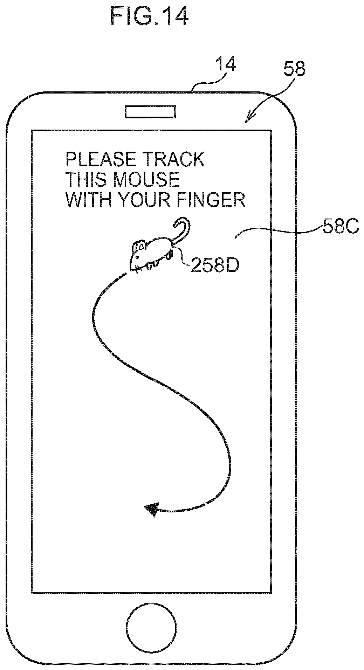

When the emotion input reception unit 260 receives the occurrence of the emotion of anger, the operation screen display control unit 262 causes the display unit 58 to display a moving object operation screen 58C illustrated in Fig. 14 and receives the input of the operation of tracking the moving object. Fig. 14 illustrates an example in which the moving object operation screen 58C displays a moving mouse mark 258D and receives the operation of tracking the moving mark 258D. Note that before the user selects the type of emotion on the emotion input screen 58B, the operation screen display control unit 262 may cause the display unit 58 to display the moving object operation screen 58C when the emotion of anger occurs in the user, and may receive the input of the operation of tracking the moving object, or after receiving the input of the operation of tracking the moving object, the operation screen display control unit may cause the emotion input reception unit 260 to receive the occurrence of the emotion of anger.

The log storage unit 62 stores the log information in association with flag information indicating whether the input regarding the occurrence of the emotion of anger by the user is received. In addition, the log storage unit 62 stores the input information of the user received regarding the occurrence of the emotion of anger in association with the log information.

In a case where the log information associated with the flag information indicating that the input from the user is not yet received is stored in the log storage unit 62, the input recommendation notification unit 64 causes the display unit 58 to display a notification for prompting the user to make an input.

In a case where there is an input instruction indicating that the user performs the input regarding the occurrence of the emotion of anger, the input reception unit 66 causes the display unit 58 to display an input screen regarding the log information, and receives the input information regarding the occurrence of the emotion of anger. The input reception unit 66 stores the received input information in the log storage unit 62 in association with the log information. At this time, the flag information for the log information is updated to flag information indicating that the input by the user is received.

On the basis of the log information and the input information stored in the log storage unit 62, the aggregation unit 68 obtains an aggregation result regarding each of the date, the time, and the place in which the emotion of anger occurs and the degree of the emotion of anger. In addition, the aggregation unit 68 obtains an aggregation result regarding each of the date, the time, and the place in which an emotion of joy occurs and the degree of the emotion of joy, and obtains the aggregation result regarding each of the date, the time, and the place in which the emotion of joy occurs and the degree of the emotion of joy.

### <Operation of Information Processing System 200>

Next, an operation of the information processing system 200 according to the second embodiment of the technology of the disclosure will be described.

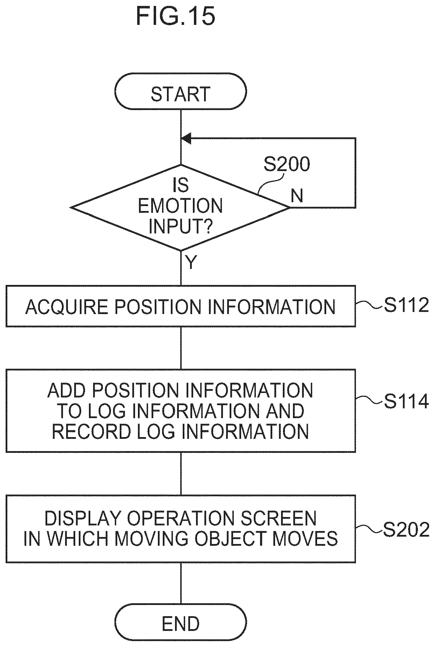

First, when the user of the mobile information terminal 214 becomes aware of the emotion of anger, the emotion of joy, or the emotion of sadness, an application for recording the log information of the mobile information terminal 214 is started. Then, when the user gives an input instruction to input the occurrence of the specific emotion to the mobile information terminal 214, the mobile information terminal 214 executes the log-information recording processing routine illustrated in Fig. 15. Note that the specific emotion is not limited to the emotion of anger, the emotion of joy, or the emotion of sadness, but includes an emotion expressing surprise, an emotion felt when receiving an impact, or the like.

First, in step S200, the emotion input reception unit 260 causes the display unit 58 to display the emotion input screen 58B, and determines whether or not an input indicating that a specific emotion occurs is received. When the type of emotion is selected on the emotion input screen 58B, log information indicating the occurrence of the selected type of emotion and the detection date and time thereof is output to the log acquisition unit 60, and the process proceeds to step S112.

In step S112, the log acquisition unit 60 acquires the current position measured by the GPS sensor 52. Then, in step S114, the log acquisition unit 60 adds the current position to the log information and stores the log information in the log storage unit 62. In addition, in a case where the type of emotion selected in step S200 is the emotion of anger, the log acquisition unit 60 stores the log information in the log storage unit 62 in association with flag information indicating that the input regarding the occurrence of the emotion of anger by the user is not received.

In step S202, in a case where the type of emotion selected in step S200 is the emotion of anger, the operation screen display control unit 262 causes the display unit 58 to display the moving object operation screen 58C and receives the input of the operation of tracking the moving object. Then, the mobile information terminal 214 ends the log-information recording processing routine. In a case where the emotion selected in step S200 is the emotion of joy or the emotion of sadness, the display on the moving object operation screen 58C is omitted.

Then, the mobile information terminal 214 periodically executes the input recommendation notification processing routine illustrated in Fig. 9.

As described above, the information processing system according to the second embodiment of the technology of the disclosure logs the occurrence of the emotional state of anger, joy, or sadness of the user, prompts the user to input the record regarding the occurrence of the emotional state of anger, and further aggregates the emotional state of anger on the basis of the log information and the input information. The information processing system can give an opportunity to objectively look back on the own specific state on the basis of the information and the aggregation result. In particular, it is possible to control an emotion by allowing the input of a cause of emotional disturbance, his/her feeling at that time, and the intensity of the emotion of anger to give the opportunity to look back.

According to the mobile information terminal, it is possible to log the occurrence of a specific emotional state of the user and receive the operation of tracking the moving object (a mouse in the example of Fig. 14) displayed on the screen from the user in the emotional state of anger, thereby distracting the emotion of anger. In particular, when the user inputs the emotion, it is possible to switch off the emotion of anger, and by the operation of tracking the moving object (the mouse in the example of Fig. 14) on the screen, it is possible to distract the mind on the spot to control the emotional disturbance.

Note that, in the first embodiment and the second embodiment described above, the mobile information terminal may display learning content, which allow the learning of the basic emotion, such as what anger is. In addition, not all the contents of the learning content can be displayed from the beginning, and the amount of the learning content that can be displayed may be increased according to the number of times of use of the application for logging the occurrence of the specific emotion of the user. For example, the amount of learning content that can be displayed may be increased according to the number of times of occurrence of the emotion of anger or the number of times of input regarding the occurrence of the emotion of anger. As described above, by gradually increasing the amount of learning content that can be displayed, it is possible to prompt the user to continuously use the application for logging the occurrence of the specific emotion of the user.

The mobile information terminal may receive an answer to a question related to anger from the user, determine how much the user is currently prone to anger, and display the determination result.

The first embodiment and the second embodiment described above may be combined. For example, the information processing system may include a wearable device that applies vibration when a button or the like is pressed at the time of feeling angry, and an information processing apparatus having a function of receiving an operation of tracking a mouse displayed on a screen at the time of receiving log information transmitted from the wearable device.

### (Supplementary note)

With regard to the above embodiments, the following supplementary notes are further disclosed.

### (Supplementary note 1)

An information processing apparatus including:
a memory; and
at least one processor connected to the memory,
in which the processor is configured to perform:
   receiving wirelessly, from a wearable device, log information indicating detection of a specific state of a wearer;
   displaying a message for prompting the wearer to make an input regarding the detection of the specific state indicated by the log information;
   receiving the input by the wearer with respect to the detection of the specific state indicated by the log information; and
   recording the log information and the input by the wearer.

### (Supplementary note 2)

A non-transitory storage medium having stored therein a program executable by a computer to execute information processing including:
receiving wirelessly, from a wearable device, log information indicating detection of a specific state of a wearer;
displaying a message for prompting the wearer to make an input regarding the detection of the specific state indicated by the log information;
receiving the input by the wearer with respect to the detection of the specific state indicated by the log information; and
recording the log information and the input by the wearer.

### (Supplementary note 3)

An information processing apparatus including:
a memory; and
at least one processor connected to the memory,
in which the processor is configured to perform:
   receiving, from a user, input information indicating that the user is in a specific state;
   displaying a message for prompting the user to make an input regarding the specific state indicated by the input information;
   receiving the input by the user with respect to the specific state indicated by the input information; and
   recording the input information and the input by the user.

### (Supplementary note 4)

A non-transitory storage medium having stored therein a program executable by a computer to execute information processing including:
receiving, from a user, input information indicating that the user is in a specific state;
displaying a message for prompting the user to make an input regarding the specific state indicated by the input information;
receiving the input by the user with respect to the specific state indicated by the input information; and
recording the input information and the input by the user.

The disclosure of Japanese Patent Application No. 2020-034124 is incorporated herein by reference in its entirety.

All documents, patent applications, and technical standards described in this specification are incorporated herein by reference to the same extent as if each individual document, patent application, and technical standard were specifically and individually described to be incorporated by reference.

## Claims

1. A wearable device comprising:
a detection unit that detects a specific state of a wearer;
a signal communication unit that wirelessly transmits, to an information processing apparatus, log information indicating a detection result obtained by the detection unit detecting the specific state; and
a stimulus application unit that applies a stimulus to the wearer when the specific state is detected by the detection unit.

2. The wearable device according to claim 1, wherein the signal communication unit transmits, to the information processing apparatus, the log information further indicating a detection date and time when the specific state is detected.

3. The wearable device according to claim 1 or 2, wherein the specific state is a specific emotional state.

4. The wearable device according to claim 3, wherein the specific emotional state is an impulsive emotion such as anger, irritation, anxiousness, panic, suicidal ideation, or catastrophic thoughts.

5. The wearable device according to claim 4, wherein the specific emotional state is an emotion of anger or anxiousness.

6. The wearable device according to any one of claims 1 to 5, further comprising:
a switch to be pressed by the wearer,
wherein the detection unit detects the specific state on a basis of pressing of the switch.

7. The wearable device according to any one of claims 1 to 6, wherein the detection unit further detects a degree of the specific state.

8. The wearable device according to any one of claims 1 to 7, wherein the wearable device is a bracelet type wearable device.

9. An information processing apparatus comprising:
a communication unit that wirelessly receives, from a wearable device, log information indicating detection of a specific state of a wearer;
a display unit that displays a message for prompting the wearer to make an input regarding the detection of the specific state indicated by the log information;
an input reception unit that receives the input by the wearer with respect to the detection of the specific state indicated by the log information; and
a recording unit that records the log information and the input by the wearer.

10. The information processing apparatus according to claim 9, wherein the communication unit receives the log information further indicating a detection date and time when the specific state is detected.

11. The information processing apparatus according to claim 9 or 10, wherein the specific state is a specific emotional state.

12. The information processing apparatus according to claim 11, wherein the specific emotional state is an impulsive emotion such as anger, irritation, anxiousness, panic, suicidal ideation, or catastrophic thoughts.

13. The information processing apparatus according to claim 12, wherein the specific emotional state is an emotion of anger or anxiousness.

14. The information processing apparatus according to claim 12 or 13, wherein the input includes a cause of occurrence of the specific emotional state or a state and a feeling of the wearer at a time of the occurrence of the specific emotional state.

15. The information processing apparatus according to any one of claims 9 to 14, wherein:
the recording unit records a detection date and time and a detection place of the specific state or a degree of the specific state, together with the log information and the input by the wearer, and
the display unit further displays a screen that visualizes an aggregation result regarding the detection date and time and the detection place of the specific state, the degree of the specific state, or the input by the wearer.

16. The information processing apparatus according to any one of claims 9 to 15, wherein the display unit further displays an operation screen for moving a moving object in the screen and receiving an operation at a time of receiving the log information.

17. An information processing system comprising:
the wearable device according to any one of claims 1 to 8; and
the information processing apparatus according to any one of claims 9 to 16,
wherein a communication unit of the information processing apparatus receives the log information from the wearable device.

18. A program for causing a computer to function as:
a communication unit that wirelessly receives, from a wearable device, log information indicating detection of a specific state of a wearer;
a display unit that displays a message for prompting the wearer to make an input regarding the detection of the specific state indicated by the log information;
an input reception unit that receives the input by the wearer with respect to the detection of the specific state indicated by the log information; and
a recording unit that records the log information and the input by the wearer.

19. The program according to claim 18, wherein the communication unit receives the log information further indicating a detection date and time when the specific state is detected.

20. An information processing apparatus comprising:
a first input reception unit that receives, from a user, input information indicating that the user is in a specific state;
a display unit that displays a message for prompting the user to make an input regarding the specific state indicated by the input information;
a second input reception unit that receives the input by the user with respect to the specific state indicated by the input information; and
a recording unit that records the input information and the input by the user.

21. The information processing apparatus according to claim 20, wherein the display unit further displays an operation screen for moving a moving object in the screen and receiving an operation when the first input reception unit receives the input information.

22. A program for causing a computer to function as:
a first input reception unit that receives, from a user, input information indicating that the user is in a specific state;
a display unit that displays a message for prompting the user to make an input regarding the specific state indicated by the input information;
a second input reception unit that receives the input by the user with respect to the specific state indicated by the input information; and
a recording unit that records the input information and the input by the user.
